(19) Europäisches Patentamt
European Patent Office
Office européen des brevets

(11) Veröffentlichungsnummer: **0 303 947**
**A2**

# (12) EUROPÄISCHE PATENTANMELDUNG

(21) Anmeldenummer: **88112952.2**

(22) Anmeldetag: **10.08.88**

(51) Int. Cl.4: **C12P 41/00 , C12P 9/00 , A01N 57/20 , C07F 9/53**

(30) Priorität: **15.08.87 DE 3727243**

(43) Veröffentlichungstag der Anmeldung: **22.02.89 Patentblatt 89/08**

(84) Benannte Vertragsstaaten: **AT BE CH DE ES FR GB IT LI NL SE**

(71) Anmelder: **HOECHST AKTIENGESELLSCHAFT**
**Postfach 80 03 20**
**D-6230 Frankfurt am Main 80(DE)**

(72) Erfinder: **Wullbrandt, Dieter, Dr.**
**Bienerstrasse 29**
**D-6238 Hofheim am Taunus(DE)**
Erfinder: **Keller, Reinhold, Dr.**
**Wiesenweg 5**
**D-6232 Bad Soden am Taunus(DE)**
Erfinder: **Schlingmann, Merten, Prof., Dr.**
**Schneidhainer Strasse 32a**
**D-6240 Königstein/Taunus(DE)**
Erfinder: **Wolfgang, Holla, Dr.**
**Am Obertor 30**
**D-6238 Hofheim am Taunus(DE)**
Erfinder: **Schneider, Manfred, Prof., Dr.**
**Triebelsheider Weg 47**
**D-5600 Wuppertal(DE)**

## (54) Verfahren zur enzymatischen Herstellung optisch aktiver phosphorhaltiger funktioneller Essigsäurederivate.

(57) Die Racematspaltung von phosphorhaltigen funktionellen Essigsäurederivaten kann mit Hilfe von Hydrolasen, wie Esterasen und Lipasen, in hoher Ausbeute und Enantiomerenreinheit durchgeführt werden.

EP 0 303 947 A2

Xerox Copy Centre

## Verfahren zur enzymatischen Herstellung optisch aktiver phosphorhaltiger funktioneller Essigsäurederivate

Die in der EP 0 106 144 und EP 0 196 026 beschriebenen racemischen phosphorhaltigen funktionellen Essigsäurederivate besitzen ausgezeichnete herbizide und wachstumsregulierende Wirkung. Wie von vielen anderen optisch aktiven Stoffen schon bekannt, zeigt auch bei diesen Verbindungen nur ein Enantiomer die beschriebene Wirkung bzw. eine stärkere Aktivität. Daher ist man natürlich daran interessiert die eigentliche Wirksubstanz aus dem racemischen Gemisch zu isolieren. Eine Trennung des in der chemischen Synthese anfallenden Racemats ist jedoch mit herkömmlichen Methoden, wie beispielsweise diastereomere Salzbildung und Kristallisation schwierig.

Die Racematspaltung proteinogener Aminosäuren mit Hilfe von Esterasen und Lipasen bzw. Proteasen ist von Whitesides [Whitesides G.M., Wong C.H., Angew. Chemie 97, 617 (1985)] beschrieben worden.

Es wurde nun gefunden, daß man das Racemat der phosphorhaltigen, funktionellen Essigsäurederivate mit Hilfe von Hydrolasen, wie Lipasen und Esterasen, auftrennen kann. Dies ist insbesondere überraschend, da diese Verbindungen in der Natur nicht vorkommen und aufgrund der Substratspezifität der verwendeten Enzyme nicht zu erwarten war, daß derartige Verbindungen erfindungsgemäß gespalten werden können.

Die Erfindung betrifft somit ein Verfahren zur Racematspaltung der Verbindung der allgemeinen Formel I,

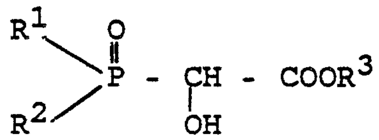

I

in der $R^1$ und $R^2$ unabhängig voneinander $C_1$ bis $C_4$ Alkyl
sowie
$R^3$ $C_1$ bis $C_{18}$ Alkyl bedeutet,
das dadurch gekennzeichnet ist, daß man die genannte Verbindung in wäßrigem oder wäßrig-organischem Medium mit einer Esterase und/oder Lipase inkubiert. Die Erfindung betrifft ferner die optisch aktive Verbindung, die durch dieses Verfahren erhältlich ist, sowie die Verwendung der Verbindung als herbizides und wachstumsregulierendes Mittel zur Bekämpfung von Unkraut und in der Landwirtschaft.

Im folgenden wird die Erfindung detailliert beschrieben, insbesondere in ihren bevorzugten Ausführungsformen. Ferner wird die Erfindung in den Patentansprüchen definiert.

Die Herstellung der Verbindufng der allgemeinen Formel I bzw. der entsprechenden Säure ist in EP 0 106 114 und EP 0 196 026 beschrieben. Die Säuren können nach herkömmlichen Methoden zu den entsprechenden Estern derivatisiert werden. Als Substrat für die enzymatische Umsetzung sind Verbindungen der allgemeinen Formel I geeignet, in der $R^1$ und $R^2$ unabhängig voneinander eine $C_1$-bis $C_4$-Alkylgruppe, bevorzugt eine $C_1$- bis $C_2$- Alkylgruppe, bedeuten können, und $R^3$ eine Alkylgruppe mit $C_1$ bis $C_{18}$ C-Atomen, bevorzugt $C_6$ bis $C_{14}$ C-Atomen ist.

Als Hydrolase, mit Hilfe derer die erfindungsgemäße Reaktion durchgeführt wird, eignen sich Lipasen oder Esterasen, wie beispielsweise mikrobielle Lipasen aus Candida cylindracea, Mucor miehei, Chromobacterium viscosum oder auch Esterasen oder Lipasen aus Schweinepankreas oder Schweineleber. Die Reaktion wird jedoch bevorzugt mit Schweineleberesterase und Candida cylindracea durchgeführt. Die Enzyme können dem Reaktionsgemisch einzeln oder zusammen zugesetzt werden.

Die erfindungsgemäß verwendete Hydrolase kann als freies, wasserlösliches Enzym oder in wasserunlöslicher Form nach herkömmlichen Methoden an einen Träger gebunden (vgl. DOS 27 32 301) in einer wäßrigen oder wäßrig-organischen Lösung eingesetzt werden. Die Substratkonzentration in der Lösung kann in weiten Bereichen schwanken. Ihr ist nach oben keine Grenze gesetzt. Bevorzugt wird in Konzentrationsbereichen von 5 bis 70 %, insbesondere 20 bis 50 % und ganz besonders bevorzugt 25 bis 40 % gearbeitet. In niedrigeren Konzentrationsbereichen kann die Reaktion ebenfalls noch, allerdings mit langsamerer Geschwindigkeit, durchgeführt werden. Als organische Lösungsmittel, die dem Reaktionsmedium zugesetzt werden können, um beispielsweise die Löslichkeit des Substrats zu erhöhen, sind mit Wasser mischbare Lösungsmittel geeignet, die die Enzymaktivität nicht nennenswert herabsetzen. Bevorzugt werden Alkohole, wie Methanol oder Ethanol, Ketone, wie Aceton oder Methylethylketon, Tetrahydrofuran, Dimethylformamid oder Dimethylsulfoxid verwendet. Die Konzentration der Lösungsmittel in der Reaktionslösung soll ebenfalls so gewählt werden, daß die Enzymaktivität nicht wesentlich herabgesetzt wird,

bevorzugt sollte sie bei ca. 5 bis 15% liegen. Die Reaktionstemperatur liegt zwischen 10 °C und 40 °C, bevorzugt zwischen 15 °C und 35 °C. Die Reaktionsdauer beträgt je nach Substrat- und Enzymkonzentration bzw. Enzymaktivität 1 bis 48 Stunden. Ausreichende enzymatische Aktivität kann bei einem pH-Wert von ca. 5 bis 9, bevorzugt bei ca. 6 bis 8, beobachtet werden. Die Reaktion kann in einem Puffer oder auch ohne Pufferzusatz durchgeführt werden. Wenn das Enzym in immobilisierter Form verwendet wird, kann dies sowohl im Batch- als auch im kontinuierlichen Verfahren geschehen.

Nach dem erfindungsgemäßen Verfahren erhält man als Endprodukt das Salz der (+)Dialkyl-phosphinoyl-hydroxyessigsäure. Der anfallende (-)Dialkyl-phosphinoyl- hydroxyessigsäureester racemisiert unter den Reaktionsbedingungen sofort zu dem (±) Ester und kann erneut der enzymatischen Spaltung unterworfen werden. Dies bedeutet, daß der (±) Ester quantitativ in das Salz der (+) Säure überführt werden kann.

Insbesondere bevorzugt wird das erfindungsgemäße Verfahren zur Racematspaltung von Dimethyl-phosphinoyl-2-hydroxyessigsäureester verwendet. Das in großer Ausbeute anfallende (+)Enantiomer hat eine hohe optische Reinheit von 85-97 % ee.

Das Endprodukt des erfindungsgemäßen Verfahrens zeigt im Vergleich zum entsprechenden Racemat die doppelte herbizide und wachstumsregulierende Aktivität. Es kann in den Anwendungsgebieten und Formulierungen eingesetzt werden, die in den Patentanmeldungen EP 0 106 114 und EP 0196 026 beschrieben wurden. Sie eignen sich sowohl zur Anwendung in der Landwirtschaft als auch zur Unkrautbekämpfung.

Die im folgenden aufgeführten Beispiele dienen zur weiteren Erläuterung der Erfindung. Die Prozentangaben beziehen sich auf das Gewicht, wenn nicht anders angegeben.

**Beispiel 1**

Zu 4,98 g (30 mmol) (±)Dimethyl-phosphinoyl-hydroxyessigsäure-methylester wurden 25 ml 0,1 molarer Natriumphosphatpuffer pH 7,0 gegeben und auf 25 °C thermostatisiert. Die Reaktion wird durch Zugabe von 5 mg Schweineleberesterase gestartet und der pH-Wert durch Zugabe 1 normaler Natronlauge konstant gehalten. Nach 140 h (88 % Umsatz der Theorie) wurde die Reaktionslösung über Celite filtriert und mit Methanol nachgewaschen. Das Lösemittel wurde im Vakuum bei maximal 40 °C entfernt, der Rückstand in Methanol aufgenommen, vom Feststoff abfiltriert und das (+)Dimethyl-phosphinoyl-hydroxyessigsäure-Natriumsalz in der Kälte zur Kristallisation gebracht.
Ausbeute:
3,9 g (+)Dimethyl-phosphinoyl-hydroxyessigsäure-Na-Salz
spezifischer Drehwert: $[\alpha]_D^{20}$ + 14,2° (c= 1,005 in Methanol)

**Beispiel 2**

Analog Beispiel 1 wurden 2,36 g (10 mmol) des (±)Hexylesters in 25 ml 0,1 molarem Natriumphosphatpuffer gelöst und durch Zugabe von 5 mg Schweineleberesterase umgesetzt. Nach 150 Stunden (50,5 % Umsatz der Theorie) bei einer Temperatur von 25 °C wurde die Reaktion abgebrochen.
Ausbeute:
0,51 g (+)Dimethyl-phosphinoyl-hydroxyessigsäure-Na-Salz (58 % der Theorie, bezogen auf das umgesetzte Produkt)
spezifischer Drehwert: $[\alpha]_D^{20}$ + 18,6° (c= 1,059 in Methanol)

**Beispiel 3**

Analog Beispiel 1 wurden 8,77 g (30 mmol) (±)Decylester in 25 ml 0,1 molarem Natriumphosphatpuffer pH 7,0 gelöst und mit 5 mg Schweineleberesterase über 19 Stunden (77 % Umsatz der Theorie) inkubiert.
Ausbeute:
3,86 g (+)Dimethyl-phosphinoyl-hydroxyessigsäure-Na-Salz (95 % der Theorie, bezogen auf das umgesetzte Produkt)
spezifischer Drehwert: $[\alpha]_D^{20}$ + 20,4° (c= 1,0746 in Methanol)
Enantiomerenreinheit: 86 % ee.

**Beispiel 4**

Analog Beispiel 1 wurden 12.0 g (41 mmol) (±) Decylester in 25 ml molarem Kaliumphosphatpuffer (pH 7.0) gelöst und mit 10 mg Schweineleberesterase über 19 Stunden inkubiert (quantitativer Umsatz).
Ausbeute:
5,84 g (+)Dimethyl-phosphinoyl-hydroxyessigsäure-K-Salz (75 % nach Umkristallisation aus Methanol).
spezifischer Drehwert von $[\alpha]_D^{20}$ + 24,1° (c = 1,0043 in Methanol)
Enantiomerenreinheit: 97 % ee.

Die Enantiomerenreinheit wurde nach diastereomerer Salzbildung mit optisch reinem 1-Phenylethylamin mittels Protonenresonanzspektroskopie bestimmt.

**Ansprüche**

1. Verfahren zur Racematspaltung der Verbindung der allgemeinen Formel I,

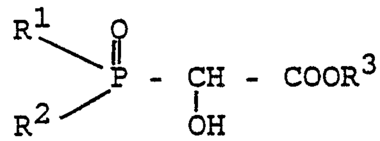

I

in der $R^1$ und $R^2$ $C_1$ bis $C_4$ Alkyl sowie
$R^3$ $C_1$ bis $C_{18}$ Alkyl bedeutet,
dadurch gekennzeichnet, daß man die genannte Verbindung in wäßrigem oder wäßrig-organischem Medium mit einer Esterase und/oder Lipase inkubiert.

2. Verfahren nach Anspruch 1, dadurch gekennzeichnet, daß mit einer Schweineleberesterase und/oder einer Lipase aus Candida cylindracea inkubiert wird.

3. Verfahren nach Anspruch 1 oder 2, dadurch gekennzeichnet, daß die Verbindung in Konzentrationen von 5 bis 70 % im Reaktionsmedium enthalten ist.

4. Verfahren nach Anspruch 3, dadurch gekennzeichnet, daß die Konzentration 20 bis 50 % beträgt.

5. Verfahren nach einem oder mehreren der Ansprüche 1 bis 4, dadurch gekennzeichnet, daß die Reaktionstemperatur zwischen 10 und 40° C liegt.

6. Verfahren nach Anspruch 5, dadurch gekennzeichnet, daß die Reaktionstemperatur 15 bis 35° C beträgt.

7. Verfahren nach einem oder mehreren der Ansprüche 1 bis 6, dadurch gekennzeichnet, daß als Verbindung (±)Dimethyl-phosphinoyl-hydroxyessigsäureester mit der allgemeinen Formel II,

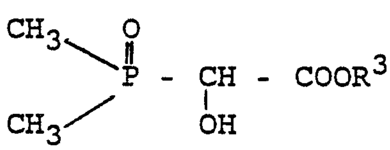

II

in der $R^3$ die in Anspruch 1 genannte Bedeutung hat, eingesetzt wird.

8. (+)Enantiomere erhältlich nach dem Verfahren nach einem oder mehreren der Ansprüche 1 bis 7.

9. (+)Dimethyl-phosphinoyl-hydroxyessigsäure der Formel III

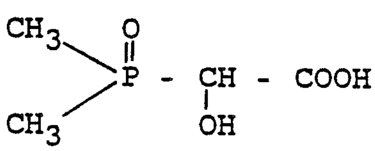

III

erhältlich nach dem Verfahren nach einem oder mehreren der Ansprüche 1 bis 7.

10. Herbizide und wachstumsregulierende Mittel, gekennzeichnet durch einen wirksamen Gehalt an einer Verbindung, erhältlich nach einem oder mehreren der Ansprüche 1 bis 7.

11. Verfahren zur Bekämpfung unerwünschten Pflanzenwuchses, dadurch gekennzeichnet, daß man auf die Pflanzen bzw. die zu behandelnden Flächen eine wirksame Menge einer Verbindung, erhältlich nach einem oder mehreren der Ansprüche 1 bis 7, aufbringt.